# EUROPEAN PATENT APPLICATION

(11) **EP 4 851 139 A1**
(43) Date of publication of application: **09.12.2026**
(21) Application number: 24938346.4
(22) Date of filing: 29.05.2024
(51) Int. Cl.: C07D 213/75, C12P 17/16, C09B 57/10, C12R 1/19, C12R 1/15, C12R 1/865, C12R 1/465

(54) **NATURAL BLUE PIGMENT AND BIOSYNTHESIS METHOD THEREFOR**

(30) Priority: 14.05.2024 CN 202410592631
(71) Applicant: Vertexyn Bioworks Co., Ltd., Nanjing, Jiangsu 210000 (CN)
(72) Inventor: SUN, Lei, Nanjing, Jiangsu 210000 (CN); XU, Fuchao, Nanjing, Jiangsu 210000 (CN); SONG, Xiaoxiao, Nanjing, Jiangsu 210000 (CN); BI, Jingjing, Nanjing, Jiangsu 210000 (CN)
(74) Representative: Chung, Hoi Kan
(86) International application number: PCT/CN2024/095947
(87) International publication number: WO 2025/236325

(57) **Abstract**

The present application relates to the technical field of biocatalysis and biosynthesis, and specifically discloses a natural blue pigment and a biosynthesis method therefor. The present application uses metabolically engineered bacteria to express indigoidine synthetase and phosphopantetheinyl transferase, to catalyze glutamine and N-acetylglutamine to biosynthesize a natural blue pigment N-acetylindigoidine, and deduces its molecular structure by means of mass spectrometry, nuclear magnetic resonance and other methods. The present application achieves catalytic synthesis of N-acetylindigoidine from glutamine and N-acetylglutamine in Escherichia coli, Corynebacterium glutamicum, Saccharomyces cerevisiae and Streptomyces. Compared with indigoidine, N-acetylindigoidine has a maximum absorption wavelength of 584 nm, possesses better color brightness, and has more stable coloration that is not easily faded, with a very broad application range and industrial production application prospects.

## Description

### TECHNICAL FIELD

The present application relates to the technical field of biocatalysis and biosynthesis, and particularly discloses a natural blue pigment and a biosynthesis method therefor.

### BACKGROUND

Indigoidine is a non-toxic natural blue product of bacteria, which is produced by catalyzing the condensation of two-molecule L-glutamine by indigoidine synthetase, i.e., non-ribosomal peptide synthase (NRPS). Indigoidine is in a bright and deep blue color that is similar to indigo, can be applied as a new natural blue pigment and dye to textile printing, food and medical industries, and has been reported to be used for dyeing of proteinaceous fiber fabrics.

Chinese Patent with the Application Publication No. of CN 109722401A discloses co-expression of an encoding gene (*bpsA)* of indigoidine synthetase from *Streptomyces lavendulae* and an encoding gene (*sfp*) of phosphopantetheinyl transferase from *Bacillus subtilis* in *Corynebacterium glutamicum*, an addition amount of a substrate (L-glutamine) is 11.68 g/L, and after continued cultivation for 48 h induced by 0.8 mM of IPTG at a condition of 18°C, a yield of indigoidine is 1.75 g/L; Chinese Patent with the Application Publication No. of CN 109722401A discloses co-expression of encoding genes of indigoidine synthetase, 4'-phosphopantetheinyl transferase and glutamine synthetase in Escherichia coli, L-glutamate is used as a substrate, indigoidine is prepared by biotransformation, and after 24 h of transformation, a yield of indigoidine reaches 5.38 g/L; Wehrs, M., et al. integrated an encoding gene (*bpsA*) of indigoidine synthetase derived from *S. lavendulae* and an encoding gene (*sfp*) of phosphopantetheinyl transferase derived from *B. subtilis* into a Saccharomyces cerevisiae genome, glucose is used as a substrate to be cultivated in a 2 L fermenter for 72 h, and a yield of indigoidine reaches 980 mg/L (Production efficiency of the bacterial non-ribosomal peptide indigoidine relies on the respiratory metabolic state in S. cerevisiae. Microb. Cell Fact. 2018, 17, No.193). Ming Zhao et al. established a highly efficient expression regulation system in Streptomyces lividans to precisely regulate an indigoidine synthetase encoding gene (*indC*) derived from Streptomyces albus J1704, glycerol is used as a substrate to be cultivated on a 4 L fermenter for 72 h, and a yield of indigoidine reaches 46.27 g/L (Establishment of an Efficient Expression and Regulation System in Streptomyces for Economical and High-Level Production of the Natural Blue Pigment Indigoidine. J Agric Food Chem Journal of Agricultural and Food Chemistry 2024 72(1), 483-492).

The above studies achieved biosynthesis of indigoidine using indigoidine synthetase from different sources and different substrates in Corynebacterium glutamicum, Escherichia coli, *Saccharomyces* and Streptomyces lividans, respectively, but no N-acetyl-indigoidine was found in the above documents.

### SUMMARY

An objective of the present application is to overcome the above-mentioned shortcomings of the prior art to provide a natural blue pigment and a biosynthesis method therefor. The natural blue pigment (N-acetyl-indigoidine) obtained in the present application has reduced polarity and water solubility compared to indigoidine due to a protective effect of acetyl, is not easily faded, is brighter in color, and has a very broad application range and industrial production prospects.

To achieve the above objective, the technical solutions adopted by the present application are as follows:

The present application provides a natural blue pigment, where the natural blue pigment has a chemical name of N-acetyl-indigoidine, a molecular formula of C₁₂H₁₀N₄O₅, and a chemical structure as shown in formula I;

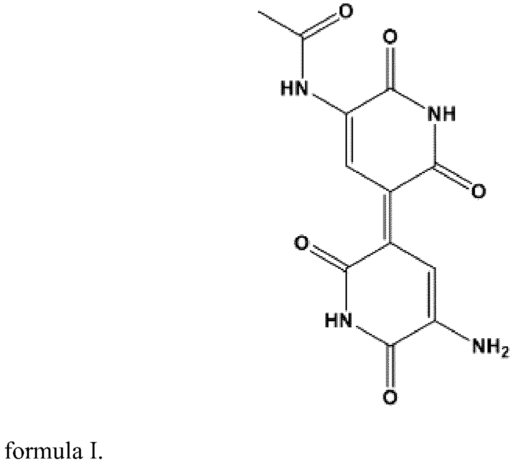

In the present application, a natural blue pigment (N-acetyl-indigoidine) of the present application is newly obtained, its molecular structure is deduced by means of mass spectrometry, nuclear magnetic resonance and other methods as shown in formula I, the molecular formula is C₁₂H₁₀N₄O₅, a relative molecular mass is 289.9, a maximum absorption wavelength is 584 nm, and characteristic peaks of -C=O and -CH₃ on acetyl appear in δ=172.23 ppm and δ=24.90 ppm of ¹³C CPMAS nuclear magnetic resonance spectra, respectively, and the color is more vivid and bright than that of indigoidine.

Amino and acetyl in N-acetyl-indigoidine forms an amide structure, reducing the polarity and hydrophilicity of N-acetyl-indigoidine. Compared with indigoidine, N-acetyl-indigoidine is more stable, is not easily faded, meanwhile, obtains better color brightness, and has a very broad application range and industrial production prospects.

The present application also provides a biosynthesis method for the natural blue pigment described above, where the biosynthesis method uses metabolically engineered bacteria to express indigoidine synthetase and phosphopantetheinyl transferase, to catalyze glutamine and N-acetylglutamine to biosynthesize the natural blue pigment.

It has been found in the present application through a large number of experiments that by using metabolically engineered bacteria to express indigoidine synthetase and phosphopantetheinyl transferase to catalyze a mixture of glutamine and N-acetyl glutamine as a substrate, the natural blue pigment (N-acetyl-indigoidine) can be biosynthesized, while the N-acetyl-indigoidine cannot be synthesized by metabolically engineered bacteria using glutamine alone or N-acetylglutamine alone as a substrate.

As a preferred embodiment of the biosynthesis method for the natural blue pigment of the present application, an encoding gene of the indigoidine synthetase includes an indigoidine synthetase encoding gene *bpsA* or an indigoidine synthetase encoding gene *indC*;
an encoding gene of the phosphopantetheinyl transferase includes a phosphopantetheinyl transferase encoding gene *EntD* or a phosphopantetheinyl transferase encoding gene *indB*.

It has been newly found in the present application that a mixture of glutamine and N-acetylglutamine can be used as a substrate to synthesize a new compound N-acetyl-indigoidine by using the metabolically engineered bacteria containing the indigoidine synthetase encoding gene *bpsA* and the phosphopantetheinyl transferase encoding gene *EntD*. It can be known through experiments that N-acetyl-indigoidine cannot be synthesized by the metabolically engineered bacteria using glutamine alone or N-acetylglutamine alone as a substrate.

According to the present application, a mixture of glutamine and N-acetylglutamine can also be used as a substrate to synthesize a new compound N-acetyl-indigoidine by introducing the indigoidine synthetase encoding gene *indC* and the phosphopantetheinyl transferase encoding gene *indB* into host bacteria.

In some specific examples, a nucleotide sequence of the indigoidine synthetase encoding gene *bpsA* is as shown in SEQ ID NO: 1, a nucleotide sequence of the indigoidine synthetase encoding gene *indC* is as shown in SEQ ID NO: 2, a nucleotide sequence of the phosphopantetheinyl transferase encoding gene *EntD* is as shown in SEQ ID NO: 3, and a nucleotide sequence of the phosphopantetheinyl transferase encoding gene *indB* is as shown in SEQ ID NO: 4.

The indigoidine synthetase and the phosphopantetheinyl transferase referred to in the present application may be derived from any species, for example, the indigoidine synthetase is derived from S*treptomyces lavendulae,* and the nucleotide sequence of the encoding gene *bpsA* of the indigoidine synthetase is as shown in SEQ ID NO: 1; or the indigoidine synthetase is derived from S*treptomyces chromofuscus* ATCC49982, and the nucleotide sequence of the encoding gene *indC* of the indigoidine synthetase is as shown in SEQ ID NO: 2. For example, the phosphopantetheinyl transferase is derived from C*orynebacterium glutamicum,* and the nucleotide sequence of the encoding gene *entD* of the phosphopantetheinyl transferase is as shown in SEQ ID NO: 3; or the phosphopantetheinyl transferase is derived from *Streptomyces chromofuscus* ATCC49982, and the nucleotide sequence of the encoding gene *indC* of the phosphopantetheinyl transferase is as shown in SEQ ID NO: 4; the above are merely examples, and the indigoidine synthetase and the phosphopantetheinyl transferase from different sources are within the scope of the present application.

As a preferred embodiment of the biosynthesis method for the natural blue pigment according to the present application, the metabolically engineered bacteria are:
metabolically engineered bacteria constructed by introducing the indigoidine synthetase encoding gene *bpsA* and the phosphopantetheinyl transferase encoding gene *EntD* into host bacteria;
   or,
metabolically engineered bacteria constructed by introducing the indigoidine synthetase encoding gene *indC* and the phosphopantetheinyl transferase encoding gene *indB* into host bacteria.

As a preferred embodiment of the biosynthesis method for the natural blue pigment according to the present application, a method for constructing the metabolically engineered bacteria includes the following steps:
subjecting the indigoidine synthetase encoding gene *bpsA* and the phosphopantetheinyl transferase encoding gene *EntD* to PCR amplification and ligation into plasmids, respectively, to obtain the metabolically engineered bacteria;
or, subjecting the indigoidine synthetase encoding gene *indC* and the phosphopantetheinyl transferase encoding gene *indB* to PCR amplification and ligation into plasmids, respectively, to obtain the metabolically engineered bacteria.

In some specific examples, the metabolically engineered bacteria are Escherichia coli recombinant strain HG-N-Idg01, and the Escherichia coli recombinant strain HG-N-Idg01 is constructed by introducing an expression vector pCDFDuet-*bpsA*-*entD* into Escherichia coli BL21(DE3).

The expression vector pCDFDuet*-bpsA-entD* is obtained by subjecting the indigoidine synthetase encoding gene *bpsA* derived from *S. lavendulae* and the phosphopantetheinyl transferase encoding gene *EntD* derived from *C. glutamicum* to PCR amplification, gene synthesis, and ligation to the expression vector pCDFDuet-1, respectively;
a method for constructing an expression vector pCDFDuet-*bpsA*-*entD* is specifically as follows:
(1) The indigoidine synthetase encoding gene *bpsA* and the phosphopantetheinyl transferase encoding gene *entD* are obtained by gene synthesis by Tsingke Biotech Co., Ltd.
(2) *bpsA* and *entD* gene fragments are sequentially inserted into two multiple cloning sites of a pCDFDuet-1 plasmid using a Seamless Cloning Kit from Takara Bio Inc. to obtain a recombinant plasmid pCDFDuet-*bpsA-entD.*
(3) pCDFDuet-*bpsA*-*entD* is transferred to Escherichia coli BL21(DE3) using a chemical transformation method to obtain an Escherichia coli recombinant strain HG-N-Idg-01.

In some specific examples, the metabolically engineered bacteria are Escherichia coli recombinant strains HG-N-Idg02 that overexpress the indigoidine synthetase encoding gene (*indC*) and the phosphopantetheinyl transferase encoding gene (*indB*) derived from *S. chromofuscus* ATCC49982, and the recombinant strains HG-N-Idg02 are constructed by introducing the expression vector pCDFDuet-*indC-indB* into Escherichia coli BL21(DE3).

The expression vector pCDFDuet-*indC*-*entD* is obtained by subjecting the indigoidine synthetase encoding gene *indC* and the phosphopantetheinyl transferase encoding gene *indB* to PCR amplification, gene synthesis, and ligation to the expression vector pCDFDuet-1, respectively.

In some specific examples, the metabolically engineered bacteria are one of a Corynebacterium glutamicum (*C. glutamicum*) recombinant strain HG-N-Idg03, a Saccharomyces cerevisiae (*S. cerevisiae*) recombinant strain HG-N-Idg04, and a Streptomyces lividans (*S. lividans*) recombinant strain HG-N-Idg05.

Among them, the Corynebacterium glutamicum (*C. glutamicum*) recombinant strain HG-N-Idg03 is constructed by introducing the expression vector pXMJ19-*bpsA*-*entD* into the Corynebacterium glutamicum ATCC13032.

The expression vector pXMJ19-*bpsA*-*entD* is obtained by subjecting the indigoidine synthetase encoding gene *bpsA* and the phosphopantetheinyl transferase encoding gene *EntD* to PCR amplification and ligation to the expression vector pXMJ19, respectively;
a method for constructing the expression vector pXMJ19-*bpsA*-*entD* is specifically as follows:
(1) The indigoidine synthetase encoding gene *bpsA* and the phosphopantetheinyl transferase encoding gene *EntD* are obtained by PCR amplification using the plasmid pCDF-*bpsA-entD* as a template.
(2) *bpsA* and *entD* gene fragments are inserted into multiple cloning sites of a pXMJ19 plasmid using a Seamless Cloning Kit from Takara Bio Inc. to obtain a recombinant plasmid pXMJ19-*bpsA-entD.*
(3) The recombinant plasmid pXMJ19*-bpsA-entD* is transferred into the Corynebacterium glutamicum ATCC13032 using an electrotransformation method to obtain a Corynebacterium glutamicum (*C. glutamicum*) recombinant strain HG-N-Idg03.

Among them, the Saccharomyces cerevisiae (*S. cerevisiae*) recombinant strain HG-N-Idg04 is constructed by introducing the expression vector pRS425-*bpsA*-*entD* into the Saccharomyces cerevisiae INVSC1.

The expression vector pRS425-*bpsA-entD* is obtained by subjecting the indigoidine synthetase encoding gene *bpsA* derived from *S. lavendulae* and the phosphopantetheinyl transferase encoding gene *EntD* derived from *C. glutamicum* to PCR amplification, gene synthesis, and ligation to the expression vector pRS425, respectively;
a method for constructing the expression vector pRS425-*bpsA*-*entD* is specifically as follows:
(1) The indigoidine synthetase encoding gene *bpsA* and the phosphopantetheinyl transferase encoding gene *EntD* are obtained by gene synthesis by Tsingke Biotech Co., Ltd.
(2) *bpsA* and *entD* gene fragments are inserted into multiple cloning sites of a pRS425 plasmid to obtain a recombinant plasmid pRS425-*bpsA-entD.*
(3) The recombinant plasmid and pRS425-*bpsA-entD* are transferred into Saccharomyces cerevisiae INVSC1 using a lithium acetate transformation method to obtain a recombinant strain HG-N-Idg-04.

Among them, the Streptomyces lividans (S. *lividans*) recombinant strain HG-N-Idg05 is constructed by introducing an expression vector pKCH-P*kasO*-*bpsA*-*entD* into Streptomyces lividans TK24.

The expression vector pKCH-P*kasO-bpsA-entD* is obtained by subjecting a hygromycin resistance gene B(hyg^{r}), a P*kasO* promoter, an indigoidine synthetase encoding gene *bpsA* derived from *S. lavendulae*, a phosphopantetheinyl transferase encoding gene *EntD* derived from *C. glutamicum*, and a terminator to PCR amplification, gene synthesis, and ligation into a plasmid pKC1139, respectively;
a method for constructing the expression vector pKCH-P*kasO*-*bpsA*-*entD* expression vector is specifically as follows:
(1) A *PermE-SlglnA-Slarg*A gene sequence, a hygromycin B resistance gene sequence and a P*kasO*-*bpsA*-*entD* gene sequence are obtained by gene synthesis by Tsingke Biotech Co., Ltd.
(2) A hygromycin B resistance gene fragment and a *PkasO-bpsA- entD* gene fragment are inserted into multiple cloning sites of a pKC1139 plasmid to obtain a recombinant plasmid pKCH-P*kasO-bpsA-entD.*
(3) The recombinant plasmid pKCH-P*kasO-bpsA-entD* is sequentially transferred into Streptomyces lividans TK24 using a conjugal transfer method to obtain a Streptomyces lividans (*S. lividans*) recombinant strain HG-N-Idg05.

Escherichia coli (*E. coli)* recombinant strains HG-N-Idg01 and HG-N-Idg02, a Corynebacterium glutamicum (*C. glutamicum)* recombinant strain HG-N-Idg03, a Saccharomyces cerevisiae (*S. cerevisiae)* recombinant strain HG-N-Idg04, and a Streptomyces lividans (S. *lividans)* recombinant strain HG-N-Idg05 constructed in the present application efficiently express the indigoidine synthetase and the 4'-phosphopantetheinyl transferase by plasmids, all achieving the catalysis of glutamine and N-acetylglutamine as substrates to biosynthesize N-acetyl-indigoidine.

The metabolically engineered bacteria of the present application can be used as cellular catalysts to catalyze the substrates to synthesize the novel compound (N-acetyl-indigoidine).

As a preferred embodiment of the biosynthesis method for the natural blue pigment according to the present application, the plasmids I include at least one of pCDFDuet, pXMJ19, pRS425, and pKC1139 plasmids.

As a preferred embodiment of the biosynthesis method for the natural blue pigment according to the present application, the PCR amplification is performed using primers with a sequence as shown in SEQ ID NO: 5 to 30.

As a preferred embodiment of the biosynthesis method for the natural blue pigment according to the present application, the host bacteria include at least one of *Escherichia coli*, *Corynebacterium glutamicum*, *Saccharomyces cerevisiae* and S*treptomyces lividans*.

Preferably, the E*scherichia coli* includes Escherichia coli BL21(DE3); the *Corynebacterium glutamicum* includes Corynebacterium glutamicum ATCC13032; the *Saccharomyces cerevisiae* includes Saccharomyces cerevisiae INVSC1; the *Streptomyces lividans* includes Streptomyces lividans TK24.

None of the original Escherichia coli BL21(DE3), *Corynebacterium glutamicum* ATCC13032, Saccharomyces cerevisiae INVSc1, and *Streptomyces lividans* TK24 are able to use glutamine and N-acetylglutamine to synthesize N-acetyl-indigoidine, and biocatalysis of N-acetyl-indigoidine can be achieved only by introduction of relevant enzyme genes into metabolically engineered bacteria in Escherichia coli, Corynebacterium glutamicum, Saccharomyces cerevisiae, and *Streptomyces*.

As a preferred embodiment of the biosynthesis method for the natural blue pigment according to the present application, the biosynthesis method includes: collecting bacterial cells induced the cultivation of the metabolically engineered bacteria by centrifugation, resuspending the bacterial cells using a catalytic solution containing phosphate buffer, glutamine, N-acetylglutamine, obtaining a transformation solution after catalysis, and collecting the natural blue pigment in the transformation solution by centrifugation.

As a preferred embodiment of the biosynthesis method for the natural blue pigment according to the present application, a concentration of glutamine in the catalytic solution is 0.1-10 g/L, and a concentration of N-acetylglutamine is 0.1-10 g/L.

Preferably, the concentration of glutamine is 1 g/L, and the concentration of N-acetylglutamine is 1 g/L.

The present application further provides a purification method for N-acetyl-indigoidine; solid precipitate in the above catalytic solution is collected by centrifugation, N-acetyl-indigoidine in the precipitate is extracted using dimethylformamide (DMF), dimethylformamide is evaporated using freeze-vacuum drying, solid is washed sequentially using ultrapure water, methanol, ethyl acetate, and n-hexane, and freeze-vacuum drying is performed again to obtain an N-acetyl-indigoidine sample.

The present application also provides a cellular catalyst, containing the metabolically engineered bacteria described above.

In some specific examples, the present application provides a cellular catalyst containing an Escherichia coli recombinant strain HG-N-Idg01; a preparation method for the cellular catalyst specifically includes the following steps: a single colony of a recombinant strain HG-N-Idg01 is picked and transferred to 5 mL of LB medium containing 50 µg/mL of streptomycin, cultivated at 37°C, 220 rpm for 16 h, and inoculated at an inoculum size of 1% into 50 mL of ZYM medium, followed by the addition of streptomycin with a final concentration of 50 µg/mL and an inducer IPTG with a final concentration of 0.1 mM, and cultivated at 30°C, 220 rpm for 24 h, bacterial cells are collected by centrifugation, and the resulting bacterial cells are the cellular catalyst.

In some specific examples, the present application provides a cellular catalyst containing a Corynebacterium glutamicum (*C. glutamicum*) recombinant strain HG-N-Idg03; a preparation method for the cellular catalyst specifically includes the following steps: a single colony of a recombinant strain HG-N-Idg03 is picked and transferred to 5 mL of BHISG medium containing 15 µg/mL of chloramphenicol and 50 µg/L of biotin, incubated at 30°C, 220 rpm for 16 h, inoculated at an inoculum size of 1% into 50 mL of GAP medium into which chloramphenicol with a final concentration of 15 µg/mL and biotin of 50 µg/L are added, cultivated at 30°C, 220 rpm for 3 h, followed by the addition of IPTG with a final concentration of 1 mM, and continuously cultivated for 24 h, bacterial cells are collected by centrifugation, and the resulting bacterial cells are the cellular catalyst.

In some specific examples, the present application provides a cellular catalyst containing a Saccharomyces cerevisiae (*S. cerevisiae*) recombinant strain HG-N-Idg04; a preparation method for the cellular catalyst specifically includes the following steps: a single colony of recombinant Saccharomyces cerevisiae HG-N-Idg04 is picked and inoculated into 5 mL of SC-Leu liquid medium, cultivated at 30°C, 220 rpm for 24 h, and centrifuged at 4°C, 1500 g for 5 min, and supernatant is removed. The cell precipitate is resuspended with 30 mL of SC-Leu liquid medium while 10 g/L of galactose is added, and cultivation is performed at 30°C, 220 rpm for 24 h. Bacterial cells are collected by centrifugation, and the resulting bacterial cells are the cellular catalyst.

In some specific examples, the present application provides a cellular catalyst containing a *Streptomyces lividans* recombinant strain HG-N-Idg05; a preparation method for the cellular catalyst specifically includes the following steps: recombinant Streptomyces lividans HG-N-Idg06 grown on an MS medium for 4-5 days is picked with a sterile tip and inoculated into a primary flask containing 10 mL of TSB medium. After 48 h of cultivation at 28°C, 1 mL is taken and transferred to a secondary flask containing 50 mL of TSB medium, and cultivation is performed at 28°C for 72 h. Bacterial cells are collected by centrifugation and are the cellular catalyst.

The present application has the following beneficial effects compared with the prior art:

The present application provides a natural blue pigment and a biosynthesis method therefor, the natural blue pigment (N-acetyl-indigoidine) obtained in the present application has a maximum absorption wavelength of 584 nm due to the protective effect of acetyl compared with indigoidine, has reduced polarity and water solubility, is not easily faded, has a brighter color, and has a very broad application range and industrial production prospects; the present application utilizes metabolically engineered bacteria to express the indigoidine synthetase and the phosphopantetheinyl transferase to catalyze glutamine and N-acetylglutamine to biosynthesize N-acetyl-indigoidine; by concluding the structure of this substance and identifying it as a new natural blue pigment, the present application also achieves the catalysis of glutamine and N-acetylglutamine to synthesize N-acetyl-indigoidine in strains of Escherichia coli, Corynebacterium glutamicum, Saccharomyces cerevisiae, *Streptomyces*, etc.

### BRIEF DESCRIPTION OF THE DRAWINGS

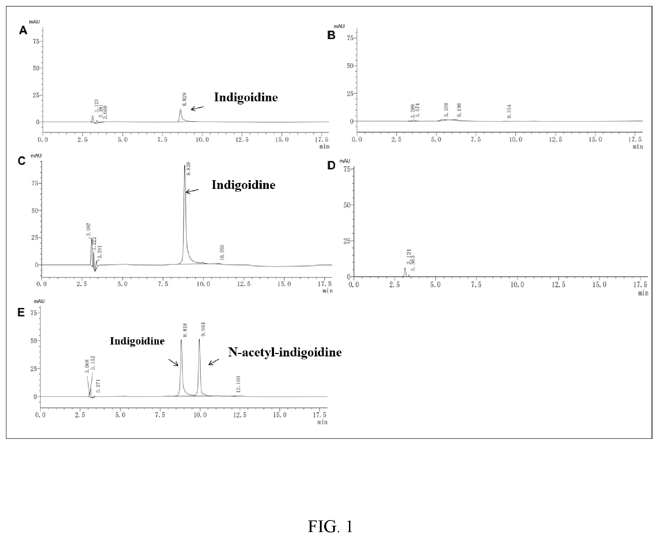

FIG. 1 is liquid chromatography of HG-N-Idg01 catalyzing different substrates:
   where (A) is a liquid chromatogram of a standard substance of indigoidine;
   (B) is a liquid chromatogram of products when a BL21(DE3) strain catalyzes a mixture of glutamine and N-acetylglutamine;
   (C) is a liquid chromatogram of products when an HG-N-Idg01 strain catalyzes glutamine;
   (D) is a liquid chromatogram of products when an HG-N-Idg01 strain catalyzes N-acetylglutamine;
   (E) is a liquid chromatogram of products when an HG-N-Idg01 strain catalyzes a mixture of glutamine and N-acetylglutamine;
FIG. 2 is an ultraviolet-visible absorption spectrogram of indigoidine (A) and N-acetyl-indigoidine (B);
FIG. 3 is an LC-MS detection diagram of N-acetyl-indigoidine;
FIG. 4 is a nuclear magnetic hydrogen spectroscopy ¹H-NMR diagram of N-acetyl-indigoidine;
FIG. 5 is a nuclear magnetic carbon spectrum ¹³C-NMR diagram of N-acetyl-indigoidine;
FIG. 6 is a nuclear magnetic HSQC spectrum of N-acetyl-indigoidine;
FIG. 7 is an HMBC spectrum of N-acetyl-indigoidine;
FIG. 8 is a speculative diagram of a structure of N-acetyl-indigoidine;
FIG. 9 is a nuclear magnetic CPMAS spectrum of indigoidine;
FIG. 10 is a nuclear magnetic CPMAS spectrum of N-acetyl-indigoidine;
FIG. 11 is a diagram showing a biosynthetic pathway of N-acetyl-indigoidine in an HG-N-Idg01 strain;
FIG. 12 is a liquid chromatogram of a catalytic product of HG-N-Idg01 (A) and HG-N-Idg02 (B);
FIG. 13 is a liquid chromatogram of a catalytic product of Corynebacterium glutamicum 13032(A) and HG-N-Idg03 (B);
FIG. 14 is a liquid chromatogram of a catalytic product of Saccharomyces cerevisiae INVSc1 (A) and HG-N-Idg04 (B);
FIG. 15 is a liquid chromatogram of a catalytic product of Streptomyces lividans TK24 (A) and HG-N-Idg05 (B);
FIG. 16 is a graph comparing colors of N-acetyl-indigoidine (A) and indigoidine (B) in DMSO;
FIG. 17 is a graph comparing colors of aqueous solutions of N-acetyl-indigoidine (A) and indigoidine (B).

### DETAILED DESCRIPTION OF THE EMBODIMENTS

For better illustrating the purposes, technical solutions and advantages of the present application, the present application will be further described below with reference to the accompanying drawings and the specific examples.

In the following examples, the experimental methods used are conventional, unless otherwise specified, and the materials, reagents, etc. used are commercially available, unless otherwise specified.

The medium components referred to in the following examples are as follows:

LB (Luria-Bertani) liquid medium: 10 g/L of peptone, 5 g/L of yeast extract, and 10 g/L of NaCl; sterilization is performed at 121°C for 20 min.

LB (Luria-Bertani) solid medium: 10 g/L of peptone, 5 g/L of yeast extract, 10 g/L of NaCl, and 15 g/L of agar powder; sterilization is performed at 121°C for 20 min, the solid medium is cooled to around 50°C, the required antibiotics is added for plate pouring, and standing is performed at 4°C after setting for standby.

ZYM fermentation medium: 96 mL of ZY medium + 2 mL of 50×M salts + 2 mL of 50×5052 + 200 µL of 1 mol/L magnesium sulfate + 100 µL of 1000× trace elements, and 20 g/L of glucose.

Among them, ZY medium: 10 g/L of peptone, and 5 g/L of yeast extract; sterilization is performed at 121°C for 20 min for standby.

Among them, 50×M salts: 1.25 mol/L of Na₂HPO₄, 1.25 mol/L of KH₂PO₄, 2.5 mol/L of NH₄Cl and 0.25 mol/L of Na₂SO₄;
among them, 50×5052: 250 g/L of glycerol, and 25 g/L of glucose; 1 mol/L of MgSO₄;
1000× trace elements: 50 mmol/L of FeCl₃, 20 mmol/L of CaCl₂, 10 mmol/L of MnCl₂, 10 mmol/L of ZnSO₄, 2 mmol/L of each of CoCl₂, NiCl₂, Na₂Mo₄, Na₂SeO₃ and H₃BO₃.
BHISG medium: 37 g/L of brain heart infusion powder, and 10 g/L of glucose;
GAP medium: 70 g/L of glucose, 40 g/L of ammonium sulfate, 1 g/L of potassium dihydrogen phosphate, 0.4 g/L of magnesium sulfate heptahydrate, 0.01 g/L of ferrous sulfate, 0.01 g/L of manganese sulfate, 4 µg/L of biotin, 200 µg/L of VB1, and 50 g/L of calcium carbonate. The pH is adjusted by KOH to 8.0, and sterilization is performed at 115°C for 30 min.

MS medium: 20 g/L of mannitol, 20 g/L pf soy flour and 15 g/L of agar.

TSB medium: 17 g/L of casein peptone, 3 g/L of soy peptone, 2.5 g/L of glucose, 5 g/L of NaCl, and 2.5 g/L of K₂HPO₄.

The primers referred to in the following examples are as shown in Table 1.

**Table 1**

| Primer Name | Sequence | Serial Number |
|---|---|---|
| pCDF-I-F1 | Gaattcgagctcggcgcgcc | SEQ ID NO:5 |
| pCDF-I-R1 | GCTAGTCTCCTGCAAGGTCATggatcctggctgtggtgatg | SEQ ID NO:6 |
| pCDF-YZ-F1 | Tgtccgggatctcgacgctc | SEQ ID NO:7 |
| pCDF-YZ-R1 | Aagcattatgcggccgcaag | SEQ ID NO:8 |
| pCDF-I-F2 | Gcgatcgctgacgtcggtac | SEQ ID NO:9 |
| pCDF-I-R2 | Ggccggccgatatccaattg | SEQ ID NO:10 |
| pCDF-YZ-F2 | Agtcgaacagaaagtaatcg | SEQ ID NO:11 |
| pCDF-YZ-R2 | Gacccgtttagaggccccaa | SEQ ID NO:12 |
| pXMJ19-I-F | gaattcagcttggctgttttg | SEQ ID NO:13 |
| pXMJ19-I-R | aagcttaattaattctgtttcctgt | SEQ ID NO:14 |
| pXMJ19-bpsA-F | gaaacagaattaattaagcttATGACCTTGCAGGAGACTAG | SEQ ID NO:15 |
| pXMJ19-bpsA-R | CATCTAATAACCCTCCTCCTTTACTCACCGAGAAG GTAAC | SEQ ID NO:16 |
| pXMJ19-entD-F | TAAAGGAGGAGGGTTATTAGatgctggatgagtctttgtt | SEQ ID NO:17 |
| pXMJ19-entD-R | caaaacagccaagctgaattctcaagtcactgcagtcgca | SEQ ID NO:18 |
| pXMJ19-YZ-R | gttccctactctcgcatggg | SEQ ID NO:19 |
| pXMJ19-YZ-F | tctggataatgttttgcg | SEQ ID NO:20 |
| pRS425-I-F | AGGTATAGCATGAGGTCGCTCggatccactagttctagagc | SEQ ID NO:21 |
| pRS425-I-R | AGAAACATTTTGAAGCTATGctcgagggggggcccggtac | SEQ ID NO:22 |
| pRS425-YZ-F | Tcccagtcacgacgttgtaa | SEQ ID NO:23 |
| pRS425-YZ-R | Ttacgccaagcgcgcaatta | SEQ ID NO:24 |
| pKCH-hyg-I-F | Tgagctcatgagcggagaacg | SEQ ID NO:25 |
| pKCH-hyg-I-R | cagtcgatcatagcacgatc | SEQ ID NO:26 |
| pKCH-hyg-YZ-F | gtctgacgctcagtggaacg | SEQ ID NO:27 |
| pKCH-hyg-YZ-R | tcatatctcattgcccccgg | SEQ ID NO:28 |
| pKCH-YZ-F | cctcttcgctattacgccag | SEQ ID NO:29 |
| pKCH-YZ-R | gagcggataacaatttcaca | SEQ ID NO:30 |

The information of strains referred to in the following examples is as shown in Table 2.

**Table 2**

| Strain Name | Strain Information |
|---|---|
| HG-N-Idg01 | *BL21(DE3)*/*pCDF-bpsA-entD* |
| HG-N-Idg02 | *BL21(DE3)*/*pCDF-indB-indC* |
| HG-N-Idg03 | ATCC13032/pXMJ19-bpsA-entD |
| HG-N-Idg04 | INVSc1/pRS425-*bpsA-entD* |
| HG-N-Idg05 | *S. lividans TK24*/*pKCH-PkasO-bpsA-entD* |

It is referred to in the present application that the indigoidine synthetase encoding gene *bpsA* is derived from *S. lavendulae;* the indigoidine synthetase encoding gene *indC* is derived from *S. chromofuscus* ATCC49982; the phosphopantetheinyl transferase encoding gene *EntD* is derived from *C*. *glutamicum;* and the phosphopantetheinyl transferase encoding gene *indB* is derived from *S. chromofuscus* ATCC49982.

The nucleotide sequence of the indigoidine synthetase encoding gene *bpsA* is as shown in SEQ ID NO: 1, the nucleotide sequence of the indigoidine synthetase encoding gene *indC* is as shown in SEQ ID NO: 2, the nucleotide sequence of the phosphopantetheinyl transferase encoding gene *EntD* is as shown in SEQ ID NO: 3, and the nucleotide sequence of the phosphopantetheinyl transferase encoding gene *indB* is as shown in SEQ ID NO: 4.

In the examples below, a high-performance liquid chromatography detection method of the sample is as follows.

### Chromatographic conditions:

Mobile phase: methanol and pure water gradient elution, with the gradient as shown in Table 3 below:

**Table 3**

| Time min | A (pure water)% | B (methyl alcohol)% |
|---|---|---|
| 0 | 80 | 20 |
| 9 | 50 | 50 |
| 13 | 80 | 20 |
| 18 | 80 | 20 |

Wavelength of 600 nm, flow rate of 1.0 ml/min, sample solution: DMSO, injection volume: 10 µL, column temperature of 35°C, run time of 18 min. Chromatographic column: Galasil^{®}EF-C18M 4.6mm id×250mm L(SN B06211801).

### Example 1. Construction of HG-N-Idg01 strain

A method for constructing an HG-N-Idg01 strain in this example includes the following steps:

### Step 1. Construction of pCDF-bpsA-entD plasmid:

(1) The indigoidine synthetase encoding gene *bpsA* (with the nucleotide sequence as shown in SEQ ID NO. 1) and the 4'-phosphopantetheinyl transferase encoding gene *entD* (with the nucleotide sequence as shown in SEQ ID NO. 3) are synthetically obtained by Tsingke Biotech Co., Ltd.
(2) PCR amplification is performed using primers pCDF-I-F1 and pCDF-I-R1 with the commercially available plasmid pCDFDuet-1 as a template, and the product is purified to obtain a pCDFDuet-1 linearized vector.
(3) The pCDFDuet-1 linearized vector and the *bpsA* gene (homology arms of the vector have been added at both ends of the gene during gene synthesis) are ligated using a Seamless Cloning Ligation Kit, and a ligation product is transferred into Escherichia coli DH5α using a chemical transformation method, and after resuscitation, plated on an LB solid medium flat plate containing 50 µg/mL of streptomycin resistance, and cultivated in an incubator at 37°C for around 16 h.
(4) Colony PCR verification is performed using primers pCDF-YZ-F1 and pCDF-YZ-R1, a strain with correct PCR verification is cultivated, the recombinant plasmid is extracted and sent to Tsingke Biotech Co., Ltd. for sequencing, and those with correct sequencing are pCDF-*bpsA* plasmids.
(5) PCR amplification is performed using pCDF-I-F2 and a pCDF-I-R2 with pCDF-*bpsA* plasmid as a template, and the product is purified to obtain a pCDF-*bpsA* linearized vector. The pCDF-*bpsA* linearized vector and a 4'-phosphopantetheinyl transferase encoding gene *entD* fragment are ligated using a Seamless Cloning Ligation Kit, and a ligation product is transferred into Escherichia coli DH5α by a chemical transformation method, and after resuscitation, plated on an LB solid medium flat plate containing 50 µg/mL of streptomycin resistance, and cultivated in an incubator at 37°C for around 16 h.
(6) Colony PCR verification is performed using primers pCDF-YZ-F2 and pCDF-YZ-R2, a strain with correct PCR verification is cultivated, the recombinant plasmid is extracted and sent to Tsingke Biotech Co., Ltd. for sequencing, and those with correct sequencing are pCDF-*bpsA-entD* plasmids (with the sequence as shown in SEQ ID NO: 31).

### Step 2. Construction of HG-N-Idg01 strain:

(1) The BL21(DE3) strain transformation competence is purchased from Tsingke Biotech Co., Ltd. under an article number of TSC-C14.
(2) The pCDF-*bpsA*-*entD* plasmids are transferred into Escherichia coli BL21(DE3) by a chemical transformation method, and after resuscitation, plated on an LB solid medium flat plate containing 50 µg/mL of streptomycin resistance, and cultivated in an incubator at 37°C for around 16 h.
(3) The single colony grown on the LB solid medium flat plate is recombinant Escherichia coli BL21(DE3)/pCDF*-bpsA-entD* and designated as HG-N-Idg01.

### Example 2. Preparation of cellular catalyst of recombinant Escherichia coli

(1) A single clone of the above recombinant Escherichia coli HG-N-Idg01 is picked and transferred to 5 mL of LB liquid medium containing 50 µg/mL of streptomycin resistance, and cultivated at 37°C, 220 rpm for 8 h.
(2) It is transferred to 50 mL of ZYM fermenter containing 50 µg/mL of streptomycin resistance and IPTG with a final concentration of 0.1 mM at an inoculum size of 1%, and cultivated at 30°C, 220 rpm for 16 h.
(3) After the end of cultivation, at 4000 rpm, 10 min, the bacterial cells are collected, and the resulting bacterial cells are the cellular catalyst.

### Example 3. Catalyzed synthesis of N-acetyl-indigoidine by different substrates

The following catalytic experiments are performed using the HG-N-Idg01 bacterial cells obtained in Example 1 as the cellular catalyst, and the results are shown in FIG. 1.

Experimental Group 1: Take 2.7 mg of standard substance of indigoidine into a 25 mL volumetric flask, add a DMSO solution to sonicate for 15 min to sufficiently dissolve, cool and set to the mark, and perform high performance liquid chromatography (HPLC) detection, with detection results as shown in FIG. 1-A.

Experimental Group 2: Collect 50 mL of fermentation broth of original Escherichia coli BL21(DE3) cultivated according to the method in Example 2 by centrifugation, resuspend the bacterial cells using 10 mL of catalytic solution (1 g/L of glutamine, 1 g/L of N-acetylglutamine, and 50 mM of PB buffer), react at 25°C, 220 rpm for 6 h, take 100 µL of sample, and perform high performance liquid chromatography detection, with detection results as shown in FIG. 1-B.

Experimental Group 3: Collect 50 mL of fermentation broth of the HG-N-Idg01 strain cultivated according to the method in Example 2 by centrifugation, resuspend the bacterial cells using 10 mL of catalytic solution (2 g/L of glutamine and 50 mM of PB buffer), react at 25°C, 220 rpm for 6 h, take 100 µL of sample, and perform high performance liquid chromatography detection, with detection results as shown in FIG. 1-C.

Experimental Group 4: Collect 50 mL of fermentation broth of the HG-N-Idg01 strain cultivated according to the method in Example 2 by centrifugation, resuspend the bacterial cells using 10 mL of catalytic solution (2 g/L of N-acetylglutamine and 50 mM of PB buffer), react at 25°C, 220 rpm for 6 h, take 100 µL of sample, and perform high performance liquid chromatography detection, with detection results as shown in FIG. 1-D.

Experimental Group 5: Collect 50 mL of fermentation broth of the HG-N-Idg01 strain cultivated according to the method in Example 2 by centrifugation, resuspend the bacterial cells using 10 mL of catalytic solution (1 g/L of glutamine, 1 g/L of N-acetylglutamine, and 50 mM of PB buffer), react at 25°C, 220 rpm for 6 h, take 100 µL of sample, and perform high performance liquid chromatography detection, with detection results as shown in FIG. 1-E.

Results are as shown in FIG. 1, and an appearance time of the standard substance of indigoidine in FIG. 1-A is around 8.8 min. There are no distinct product peaks in FIG. 1-B. There is one peak present around 8.8 min in FIG. 1-C, which is consistent with the appearance time of the standard substance of indigoidine, and a catalytic product is indigoidine. There are no distinct product peaks in FIG. 1-D. There is one peak each present around 8.8 min and around 9.9 min in FIG. 1-E each, speculating that products are indigoidine and N-acetyl-indigoidine, respectively. It is indicated that the original strain BL21(DE3) is unable to catalyze 1 g/L of glutamine and 1 g/L of N-acetylglutamine to synthesize N-acetyl-indigoidine, the recombinant Escherichia coli HG-N-Idg01 overexpresses *bpsA* and *entD* genes by plasmids to achieve catalysis of glutamine and N-acetylglutamine to synthesize N-acetyl-indigoidine, and N-acetyl-indigoidine cannot be synthesized by a single substrate of glutamine or N-acetylglutamine.

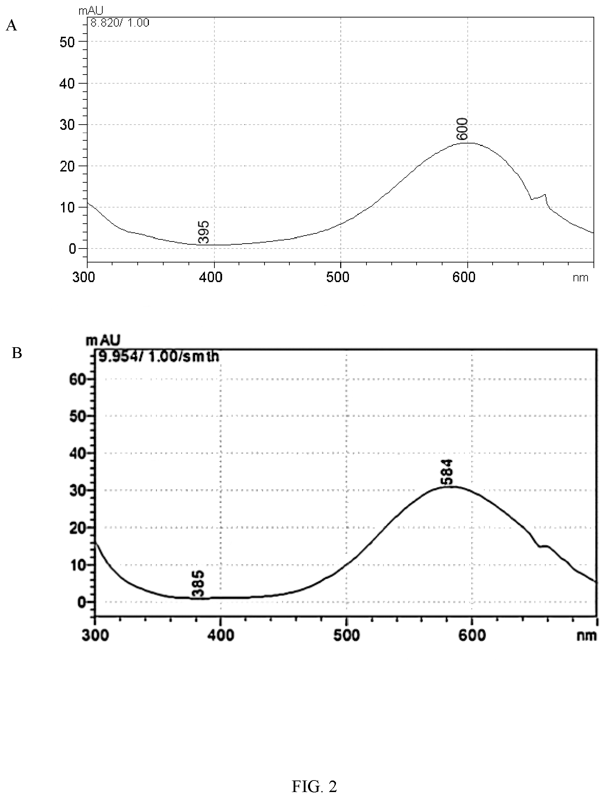

FIG. 2 shows a wavelength corresponding to the maximum absorption peak of Experimental Group 5, where the absorption peak of indigoidine at 8.8 min corresponds to a wavelength of 600 nm, as shown in FIG. 2-A; the absorption peak of N-acetyl-indigoidine at 9.9 min corresponds to a wavelength of 584 nm, as shown in FIG. 2-B.

### Example 4. Identification of structure of N-acetyl-indigoidine sample:

The catalytic reaction solution of Experimental Group 5 in Example 3 is collected and centrifuged at 10000 g for 5 min, precipitated cells are resuspended with 10 mL of dimethylformamide (DMF) after supernatant is removed, and the cells are broken by sonication, so that N-acetyl-indigoidine is extracted into a solvent. The supernatant is obtained by centrifugation again, an organic solvent is evaporated in vacuum, and then dark solid is washed twice with 10 mL of pure water, methanol, ethyl acetate and hexane, respectively; finally, vacuum freeze drying is performed to obtain 0.14 g of N-acetyl-indigoidine powder. The above samples are sent to the Analysis and Testing Center of Nanjing Normal University for LC-MS detection and NMR detection.

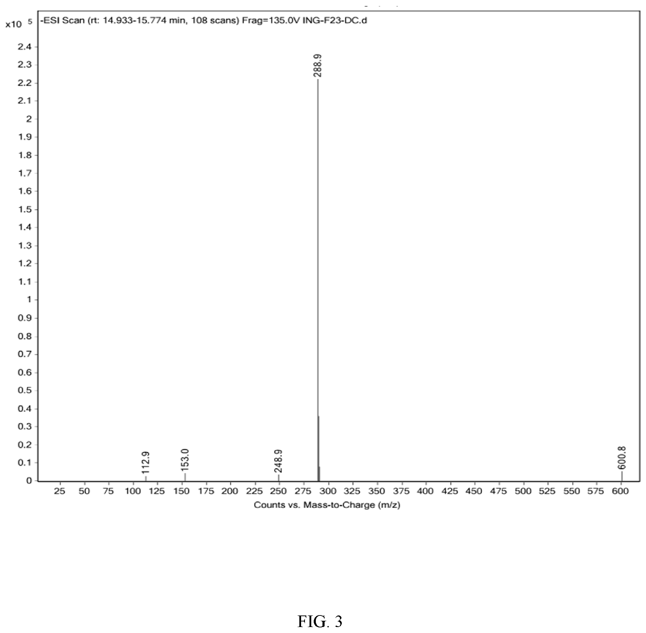

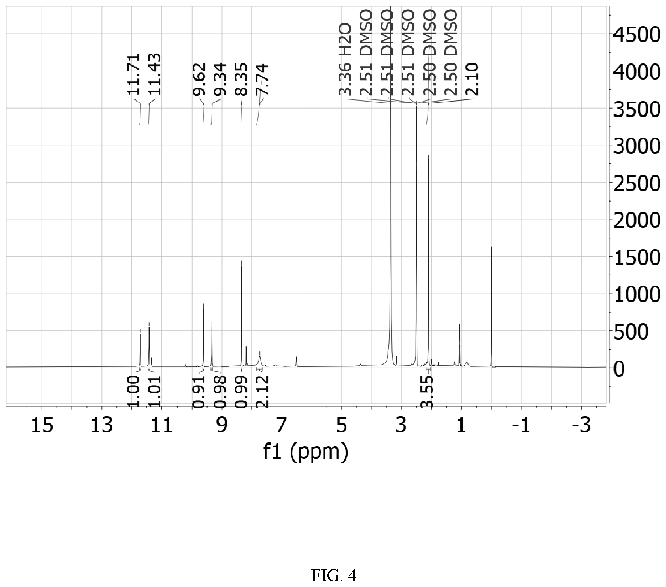

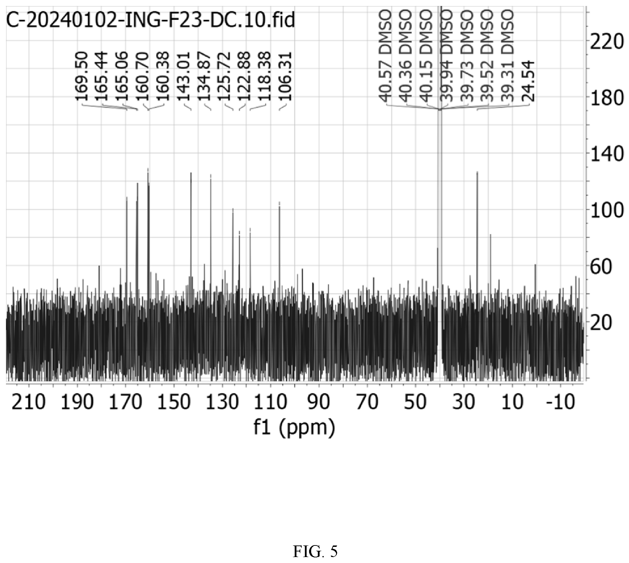

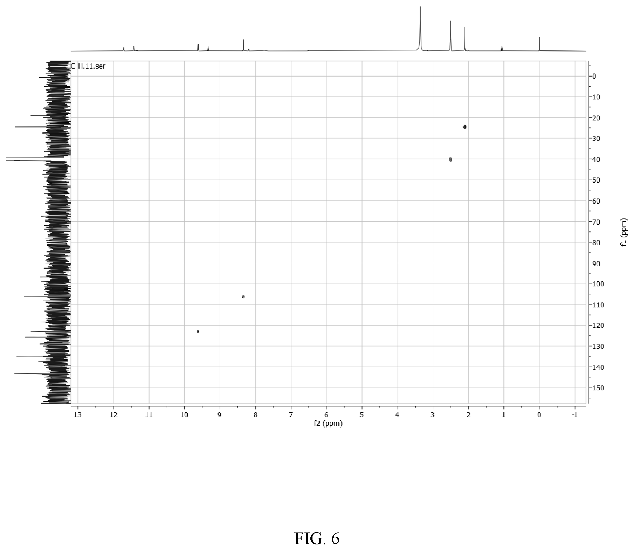

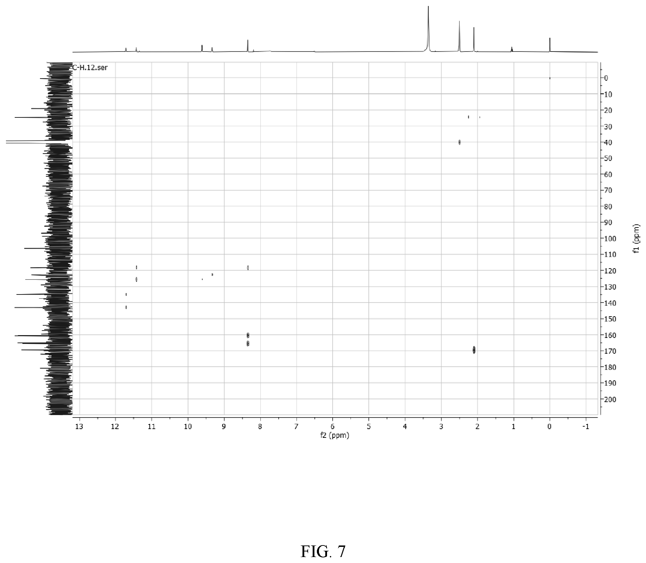

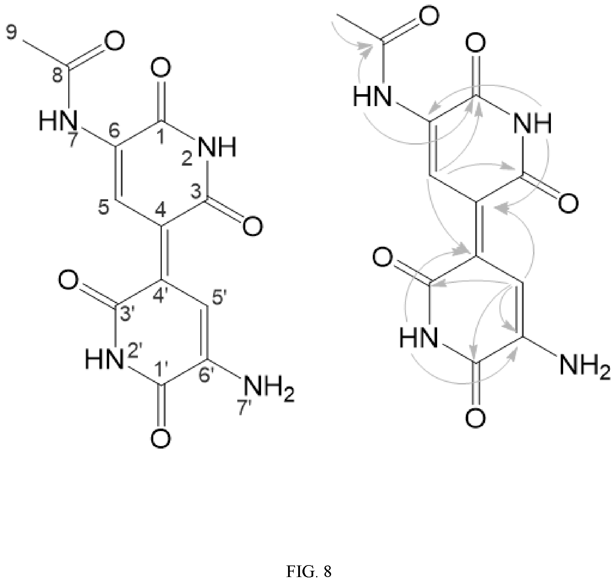

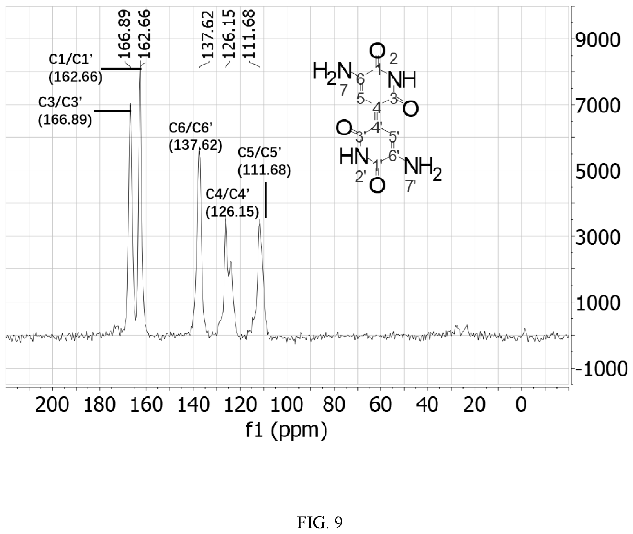

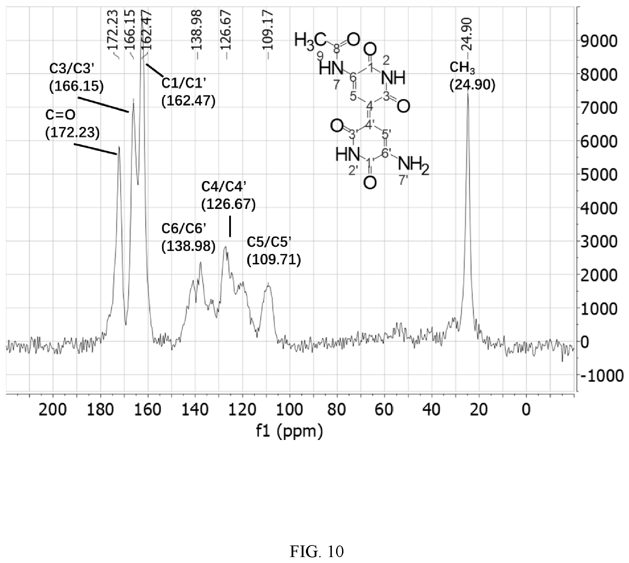

FIG. 3 is an LC-MS detection diagram; FIG. 4 is a ¹H-NMR diagram of N-acetyl-indigoidine; FIG. 5 is a ¹³C-NMR diagram of N-acetyl-indigoidine; FIGs. 6-7 are nuclear magnetic HSQC and HMBC spectra of N-acetyl-indigoidine; FIG. 8 is a speculative diagram of a structure of N-acetyl-indigoidine; FIG. 9 is a nuclear magnetic CPMAS spectrogram of indigoidine; FIG. 10 is a nuclear magnetic CPMAS spectrum of N-acetyl-indigoidine.

Table 4 shows the correspondence of data of ¹H-NMR (FIG. 4) and ¹³C-NMR (FIG. 5) with element positions in a speculated structure (FIG. 8).

**Table 4 Nuclear Magnetic Data Analysis**

| Position | *δ*_{H} | *δ*_{C} |
|---|---|---|
| 1 | | 160.38 |
| 2-NH | 11.43(1H,s) | |
| 3 | | 165.44 |
| 4 | | 134.87 |
| 5 | 8.35(1H,s) | 106.31 |
| 6 | | 143.01 |
| 7-NH | 9.34(1H,s) | |
| 8 | | 169.50 |
| 9 | 2.10(3H,s) | 24.54 |
| 1' | | 160.70 |
| 2'-NH | 11.71(1H,s) | |
| 3' | | 165.06 |
| 4' | | 118.39 |
| 5' | 9.62(1H,s) | 122.88 |
| 6' | | 125.72 |
| 7'-NH₂ | 7.74(2H,s) | |

The relative molecular mass of indigoidine is 248.19, the relative molecular mass of acetyl is 43, and LC-MS detection results (FIG. 3) show that the relative molecular mass of the sent sample is 289.9, which is consistent with a theoretical value of N-acetyl-indigoidine.

As shown in the ¹H-NMR spectrum (FIG. 4), a peak of the solvent DMSO is at the δ value around 2.50 ppm, and the water peak is at 3.36 ppm. Taking an isolated peak at 11.71 ppm as an integral standard, the product is 1. From the HSQC spectrum (FIG. 6), there is no point on the abscissa corresponding to 11.71 ppm, suggesting hydrogen on nitrogen here; 11.43 ppm, 7.74 ppm, and 9.34 ppm are similar, suggesting hydrogen on nitrogen; the peaks at 8.35 ppm and 9.62 ppm are singlets, with carbons corresponding to 100-160 ppm present on the HSQC spectrum (FIG. 6), suggesting olefinic carbon; the peak at 2.10 ppm is an s peak, and the integral is 3, suggesting the presence of one methyl. The integral of the peak at 7.74 ppm is 2, suggesting primary amine; the integral of the peaks at 11.71 ppm, 11.43 ppm, and 9.34 ppm is 1, suggesting secondary amine. It can be speculated by ¹H-NMR that the compound has one primary amine, three secondary amines, two double bonds, and one methyl.

In combination with analysis of ¹³C-NMR spectrum (FIG. 5), those with the δ value of 24.54 ppm are methyl signals; those with the δ value of 160 ppm-180 ppm are carbonyl signals, so there are five carbonyl at the δ values of 169.50 ppm, 165.44 ppm, 165.06 ppm, 160.70 ppm, and 160.38 ppm; typically, SP2 hybridized carbon atoms are at 100-150 ppm, the peaks at 122.88 ppm and 106.31 ppm are carbon corresponding to the ¹H-NMR spectrum, suggesting olefinic carbons, and the remaining 143.01 ppm, 134.87 ppm, 125.72 ppm, and 118.39 ppm are all unsaturated carbons. In view of the above, and in combination with the structural formula of indigoidine, it is speculated that the structural formula of N-acetyl-indigoidine is as shown in FIG. 8. Assignment of signals of the ¹H-NMR spectrum and ¹³C-NMR spectrum is as shown in Table 4.

It is speculated that the molecular formula of the new substance is C₁₂H₁₀N₄O₅; by comparing the nuclear magnetic CPMAS spectra (FIG. 9 and FIG. 10) of indigoidine and N-acetyl-indigoidine, the characteristic peaks of -C=O and -CH₃ on acetyl appear at δ=172.23 ppm and δ=24.90 ppm, respectively, and therefore the new substance is named N-acetyl-indigoidine. This structure has not been reported in the literature and is an entirely new substance. This new substance appears dark blue in the solution, has a maximum light absorption value of 584 nm, and is speculated to be a new blue pigment.

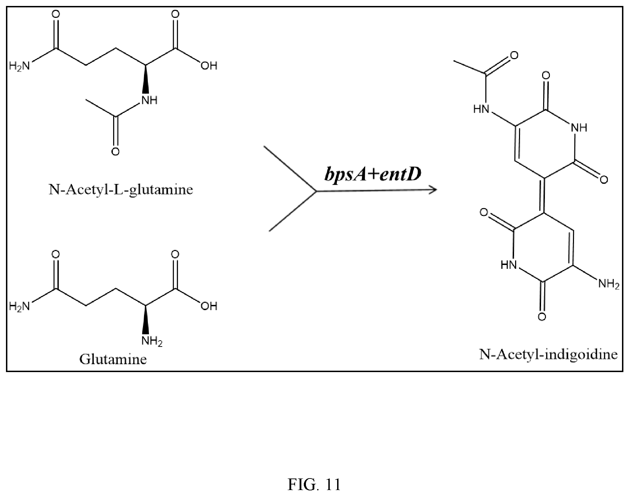

A biosynthetic pathway of N-acetyl-indigoidine in an HG-N-Idg01 strain is as shown in FIG. 11.

### Example 5. Construction of HG-N-Idg02 strain

In order to compare the biological activities of the indigoidine synthetase and the phosphopantetheinyl transferase from different species, the present application constructs an Escherichia coli recombinant strain HG-N-Idg02 overexpressing the indigoidine synthetase encoding gene (*indC*) and the phosphopantetheinyl transferase encoding gene (*indB*) derived from *S. chromofiuscus* ATCC49982.

The construction process is as follows:
Step 1. Construction of pCDF-*indB-indC* plasmid:
   (1) The indigoidine synthetase encoding gene *indC* (with the nucleotide sequence as shown in SEQ ID NO. 3) and the 4'-phosphopantetheinyl transferase encoding gene *indB* (with the nucleotide sequence as shown in SEQ ID NO. 4) are synthetically obtained by Tsingke Biotech Co., Ltd.
   (2) The pCDF-*indB-indC* plasmid is constructed according to the method of Example 1.
Step 2. Construction of HG-N-Idg02 strain:
   The pCDF*-indB-indC* constructed in step 1 is transferred into BL21(DE3) using a chemical transformation method to obtain recombinant Escherichia coli BL21(DE3)/pCDF-*indB-indC*, which is designated as HG-N-Idg02.

### Example 6. Comparison of catalytic capacity of indigoidine synthetase from different sources

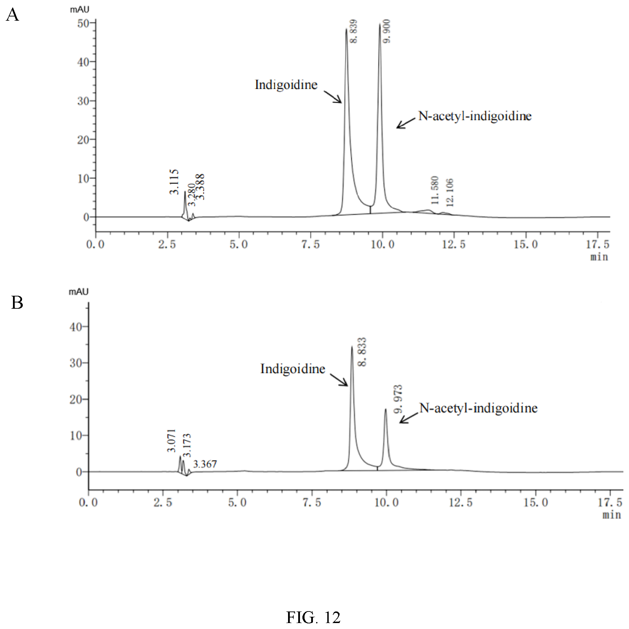

HG-N-Idg01 and HG-N-Idg02 cellular catalysts are respectively prepared using a cultivation method in Example 2, bacterial cells are respectively resuspended using 10 mL of catalytic solution (1 g/L of glutamine + 1 g/L of N-acetylglutamine + 50 mM of PB) and reacted at 20°C, 220 rpm for 6 h, and 100 µL of each is taken to be used for HPLC detection. Testing results are as shown in FIG. 12. The HG-N-Idg01 and HG-N-Idg02 catalytic reaction solutions each show two peaks at 8.8 min and 9.9 min, and peak areas of the HG-N-Idg01 catalytic products of indigoidine and N-acetyl-indigoidine are larger than that of HG-N-Idg02. The results indicate that *indB and indC* are able to catalyze glutamine and N-acetylglutamine to produce indigoidine and N-acetyl-indigoidine, but the enzymatic activity is lower than those of *bpsA* and *entD*.

According to the present application, the biosynthesis of N-acetyl-indigoidine is achieved using indigoidine synthetase and phosphopantetheinyl transferase from different sources in Escherichia coli.

### Example 7. Construction of recombinant Corynebacterium glutamicum HG-N-Idg03

This example provides a method for constructing recombinant Corynebacterium glutamicum HG-N-Idg03, including the following steps:
Step 1. Construction of pXMJ19-*bpsA-entD* plasmid:
   (1) PCR amplification is performed using primers pXMJ19-*bpsA*-F and pXMJ19-*bpsA*-R with pCDF-*bpsA-entD* plasmids constructed in Example 1 as a template to obtain a *bpsA* gene fragment; PCR amplification is performed using pXMJ19-*entD*-F and pXMJ19-*entD*-R as primers to obtain *entD* gene fragments;
   (2) PCR amplification is performed using primers pXMJ19-I-F and pXMJ19-I-R with the commercially available plasmid pXMJ19 as a template to obtain a pXMJ19 linearized vector.
   (3) Ligation is performed using a Seamless Cloning Ligation Kit of Takara Bio Inc., and a ligation product is transferred into Escherichia coli DH5α, and after resuscitation, plated on an LB solid medium flat plate containing 15 µg/mL of chloramphenicol resistance, and cultivated in an incubator at 37°C for around 16 h.
   (4) Colony PCR verification is performed using primers pXMJ19-YZ-F and pXMJ19-YZ-R, a strain with correct PCR verification is cultivated, the recombinant plasmid is extracted and sent to Tsingke Biotech Co., Ltd. for sequencing, and those with correct sequencing are pXMJ19-*bpsA-entD* plasmids.
Step 2. Preparation of ATCC13032 electrotransformation competence:
   (1) A single colony of ATCC13032 is picked into 5 mL of antibiotic-free BHISG medium, and cultivated at 30°C, 220 rpm overnight;
   (2) after measuring OD₆₀₀, 150D bacterial solution is taken to be inoculated in 50 mL of BHISGGT medium (0.1% Tween 80 and 50 µg/L of biotin), cultivated at 30°C, 220 rpm until OD₆₀₀ is about 1.0, taken out and placed on ice, and subjected to an ice bath for 20 min;
   (3) the bacterial solution is transferred to a 50 ml centrifuge tube and centrifuged at 4°C, 2600×g for 10 min, supernatant is discarded, and the residual bacterial solution is aspirated with a gun;
   (4) the bacterial cells are resuspended by adding 50 mL of 10% glycerol (pre-chilled) and centrifuged at 4°C, 2600×g for 10 min, supernatant is discarded, and the residual bacterial solution is aspirated with a gun; this step is repeated once;
   (5) the bacterial cells are resuspended with 100 µL of 10% glycerol (pre-chilled) and transferred to a 1.5 mL EP tube for standby.
Step 3. Construction of recombinant strain HG-N-Idg03:
   (1) 200 ng of pXMJ19-*bpsA*-*entD* plasmids are taken and added into electrotransformation competence cells prepared in step 2, and they are mixed well gently, then added into an electroporation cuvette, and subjected to an ice bath for 5-10 min;
   (2) after 2-3 1.8 KV electric shocks, the pre-warmed BHISG medium at 46°C is rapidly added, heat shocking is performed in a water bath at 46°C for 6 minutes, and then the heat-shocked transformation solution is incubated in a shaking table at 30°C for 2 hours;
   (3) after the end of incubation, 1 mL of transformation solution is taken and centrifuged at 4000 rpm, most of the supernatant is discarded, and the remaining supernatant is resuspended, plated on a chloramphenicol-resistant BHISG flat plate, and cultivated in an incubator at 30°C for 48 h.
   (4) The single clone grown is recombinant Corynebacterium glutamicum HG-N-Idg03.

### Example 8. Catalytic synthesis of N-acetyl-indigoidine by recombinant Corynebacterium glutamicum HG-N-Idg03

A single colony of a recombinant Corynebacterium glutamicum strain HG-N-Idg03 is picked and transferred to 5 mL of BHISG medium containing 15 µg/mL of chloramphenicol and 50 µg/L of biotin, cultivated at 30°C, 220 rpm for 16 h, inoculated at an inoculum size of 1% into 50 mL of GAP medium in which chloramphenicol with a final concentration of 15 µg/mL and biotin with a final concentration of 50 µg/L are added, cultivated at 30°C, 220 rpm for 3 h, followed by the addition of IPTG with a final concentration of 1 mM, and continuously cultivated for 24 h, bacterial cells are collected by centrifugation to serve as the cellular catalyst.

The bacterial cells are resuspended using 10 mL of catalytic reaction solution (1 g/L of glutamine, 1 g/L of N-acetylglutamine, and 50 mM of PB) and reacted at 25°C, 220 rpm for 6 h, and 100 µL of sample is taken for HPLC detection. The original strain Corynebacterium glutamicum ATCC13032 is used as a control group.

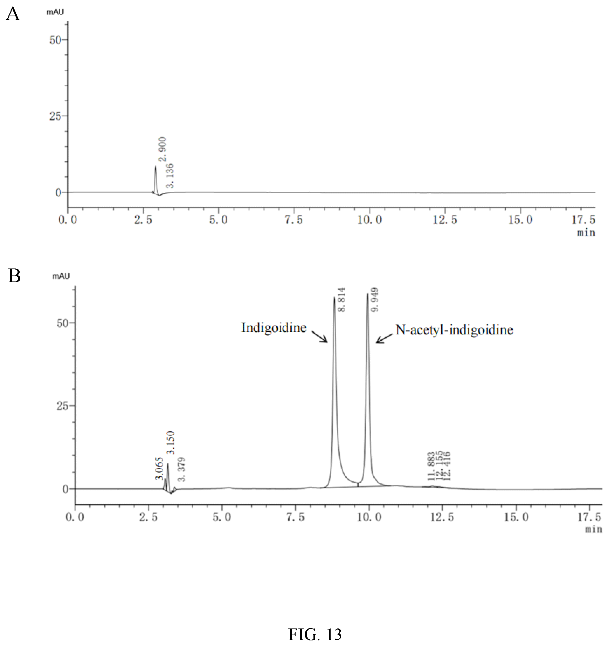

Testing results are as shown in FIG. 13; FIG. 13-A shows testing results of the ATCC13032 strain, and there are no significant peaks at 8.8 min and 9.9 min; FIG. 13-B shows testing results of the HG-N-Idg03 strain, with one peak each present at 8.8 min and 9.9 min, which is consistent with the appearance time of indigoidine and N-acetyl-indigoidine, respectively. It is indicated that the original strain ATCC13032 is unable to catalyze glutamine and N-acetyl-indigoidine to synthesize N-acetyl-indigoidine, the recombinant Corynebacterium glutamicum strain HG-N-Idg03 overexpresses *bpsA* and *entD* genes by plasmids to achieve catalysis of glutamine and N-acetylglutamine to synthesize N-acetyl-indigoidine.

### Example 9. Construction of recombinant Saccharomyces cerevisiae HG-N-Idg04

This example provides a method for constructing recombinant Saccharomyces cerevisiae HG-N-Idg04, including the following steps:
Step 1. Construction of pRS425-*bpsA-entD* plasmid:
   (1) The indigoidine synthetase encoding gene *bpsA* is synthesized by Tsingke Biotech Co., Ltd., promoter (TEF1 promoter) and terminator (CYC1 terminator) sequences are introduced during synthesis, and a nucleotide sequence of a TEF1 promoter-*bpsA-*CYC1 terminator expression cassette is as shown in SEQ ID NO: 32.
   (2) The 4'-phosphopantetheinyl transferase encoding gene *entD* is synthesized by Tsingke Biotech Co., Ltd., promoter (TEF1 promoter) and terminator (ADH1 terminator) sequences are introduced during synthesis, and a nucleotide sequence of a TEF1 promoter-*entD*-ADH1 terminator expression cassette is as shown in SEQ ID NO: 33.
   (3) PCR amplification is performed using primers pRS425-I-F and pRS425-I-R with the commercially available pRS425 plasmid as a template to obtain a pRS425 linearized vector.
   (4) The pRS425 linearized vector and the TEF1 promoter-*bpsA-*CYC1 terminator as well as the TEF1 promoter-*entD*-ADH1 terminator are ligated using a Seamless Cloning Ligation Kit, and a ligation product is transferred into Escherichia coli DH5α using a chemical transformation method, and after resuscitation, plated on an LB solid medium flat plate containing 100 µg/mL of ampicillin resistance, and cultivated in an incubator at 37°C for around 16 h.
   (5) Colony PCR verification is performed using primers pRS425-YZ-F and pRS425-YZ-R, a strain with correct PCR verification is cultivated, the recombinant plasmid is extracted and sent to Tsingke Biotech Co., Ltd. for sequencing, and those with correct sequencing are pRS425-*bpsA-entD* plasmids.
Step 2. Construction of HG-N-Idg04 strain:

The recombinant plasmid pRS425-*bpsA-entD* is transformed into Saccharomyces cerevisiae INVSc1 by a lithium acetate transformation method, and after resuscitation, plated on an SC-Leu selective medium and cultivated at 30°C for 48 h; a single colony grown is recombinant Saccharomyces cerevisiae INVSc1/pRS425-*bpsA*-*entD*, which is designated as recombinant Saccharomyces cerevisiae HG-N-Idg04.

### Example 10. Catalytic synthesis of N-acetyl-indigoidine by recombinant Saccharomyces cerevisiae HG-N-Idg04

A single colony of recombinant Saccharomyces cerevisiae HG-N-Idg04 is picked and inoculated into 5 mL of SC liquid medium, and cultivated at 30°C, 220 rpm for 24 h. Centrifugation is performed at 4°C, 1500 g for 5 min, and supernatant is removed. The cell precipitate is resuspended with 50 mL of SC-Leu liquid medium while 10 g/L of galactose is added, and is cultivated at 30°C, 220 rpm for 24 h. Bacterial cells are collected by centrifugation and are the HG-N-Idg04 cellular catalyst. The bacterial cells are resuspended using 10 mL of catalytic solution (1 g/L of glutamine, 1 g/L of N-acetylglutamine, and 50 mM of PB) and reacted at 25°C, 220 rpm for 6 h, and 100 µL of sample is taken for HPLC detection. The control group is the original strain INVSc1.

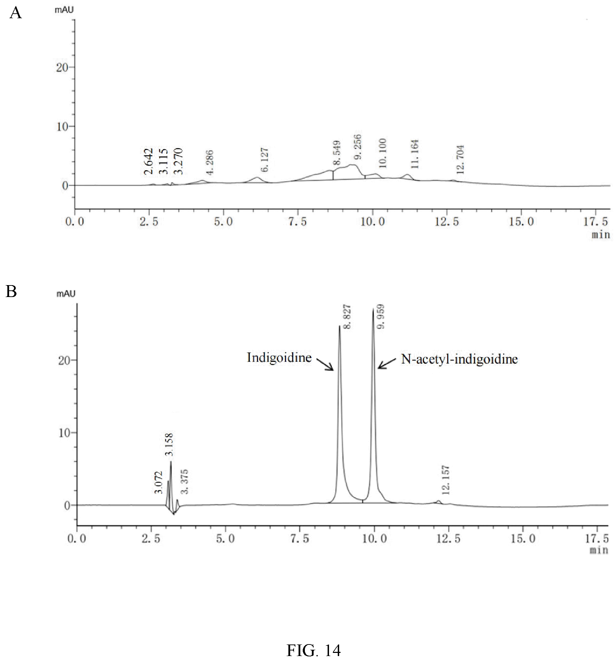

Testing results are as shown in FIG. 14; FIG. 14-A shows testing results of the INVSc1 strain, and there are no significant peaks at 8.8 min and 9.9 min; FIG. 14-B shows testing results of the HG-N-Idg04 strain, with one peak each present at 8.8 min and 9.9 min, which is consistent with the appearance time of indigoidine and N-acetyl-indigoidine, respectively. It is indicated that the original strain INVSc1 is unable to catalyze glutamine and N-acetyl-indigoidine to synthesize N-acetyl-indigoidine, the recombinant Saccharomyces cerevisiae strain HG-N-Idg04 overexpresses *bpsA* and *entD* genes by plasmids to achieve catalysis of glutamine and N-acetylglutamine to synthesize N-acetyl-indigoidine.

### Example 11. Construction of recombinant Streptomyces lividans HG-N-Idg05

This example provides a method for constructing recombinant Streptomyces lividans HG-N-Idg05, including the following steps:
Step 1. Construction of hygromycin B resistance plasmid pKC-hyg:
   (1) PCR amplification is performed using primers pKCH-hyg-I-F and pKCH-hyg-I-R with the commercially available plasmid pKC1139 as a template to obtain a pKC1139 linearized vector;
   (2) the hygromycin B resistance gene (hyg^{r}) is synthesized by Tsingke Biotech Co., Ltd., and the nucleotide sequence is as shown in SEQ ID NO: 34.
   (3) The pKC1139 linearized vector and the hygromycin B resistance gene fragment are ligated using a Seamless Cloning Ligation Kit of Takara Bio Inc., and a ligation product is transferred into Escherichia coli DH5α, and after resuscitation, plated on an LB solid medium flat plate containing 100 µg/mL of hygromycin B resistance, and cultivated in an incubator at 37°C for around 16 h.
   (4) Colony PCR verification is performed using primers pKC-hyg-YZ-F and pKC-hyg-YZ-R, a strain with correct PCR verification is cultivated, the recombinant plasmid is extracted and sent to Tsingke Biotech Co., Ltd. for sequencing, and those with correct sequencing are hygromycin B resistance plasmids pKC-hyg.
Step 2. Construction of pKCH-P*kasO-bpsA-entD* plasmid:
   (1) PCR amplification is performed using primers pKCH-I-F and pKCH-I-R with the pKC-hyg plasmid constructed in step 1 as a template to obtain a pKCH-hyg linearized vector.
   (2) The *PkasO-bpsA-entD* expression cassette gene sequence is synthesized by Tsingke Biotech Co., Ltd., and a nucleotide sequence is as shown in SEQID NO: 35.
   (3) The pKCH-hyg linearized vector and the P*kasO*-*bpsA*-*entD* are ligated using a Seamless Cloning Ligation Kit of Takara Bio Inc., and a ligation product is transferred into Escherichia coli DH5α, and after resuscitation, plated on an LB solid medium flat plate containing 100 µg/mL of hygromycin B resistance, and cultivated in an incubator at 37°C for around 16 h.
   (4) Colony PCR verification is performed using primers pKCH-YZ-F and pKCH-YZ-R, a strain with correct PCR verification is cultivated, the recombinant plasmid is extracted and sent to Tsingke Biotech Co., Ltd. for sequencing, and those with correct sequencing are pKCH-P*kasO*-*bpsA -entD* plasmids.
Step 3. Construction of recombinant strain HG-N-Idg05:
   Streptomyces lividans TK24 and *E. coli* ET12567/pUZ8002 are purchased from Biofeng. The recombinant plasmid pKCH-P*kasO*-*bpsA*-*entD* is transformed into Streptomyces lividans TK24 by adopting a conjugal transfer method to further construct HG-N-Idg05.

A specific experimental process is as follows:
(1) Plasmid pKCH-P*kasO-bpsA-entD* for conjugal transfer is transformed into *E. coli* ET12567/pUZ8002;
(2) biparental conjugal transfer is performed on *E. coli* ET12567/pUZ8002 containing this plasmid with streptomyces lividans TK24;
(3) coverage with hygromycin B and nalidixic acid is performed after 18 h of cultivation at 28°C;
(4) after 3-5 days of cultivation at 28°C, conjugative transformants are picked to extract genomic DNA;
(5) PCR verification is performed using primers pKCH-YZ-F and pKCH-YZ-R, those with correct PCR verification are recombinant Streptomyces lividans HG-N-Idg05:TK24/pKCH-P*kasO-bpsA-entD.*

### Example 12. Catalytic synthesis of N-acetyl-indigoidine by recombinant Streptomyces lividans HG-N-Idg05

Recombinant Streptomyces lividans HG-N-Idg05 grown on an MS medium for 4-5 days is picked with a sterile tip and inoculated into a primary flask containing 10 mL of TSB medium. After 48 h of cultivation at 28°C, 1 mL is taken and transferred to a secondary flask containing 50 mL of TSB medium, and cultivated at 28°C for 72 h. Bacterial cells are collected by centrifugation and are the HG-N-Idg06 cellular catalyst. The bacterial cells are resuspended using 10 mL of catalytic solution (1 g/L of glutamine, 1 g/L of N-acetylglutamine, and 50 mM of PB) and reacted at 25°C, 220 rpm for 6 h, and 100 µL of sample is taken for HPLC detection. The control group is the original strain streptomyces lividans TK24.

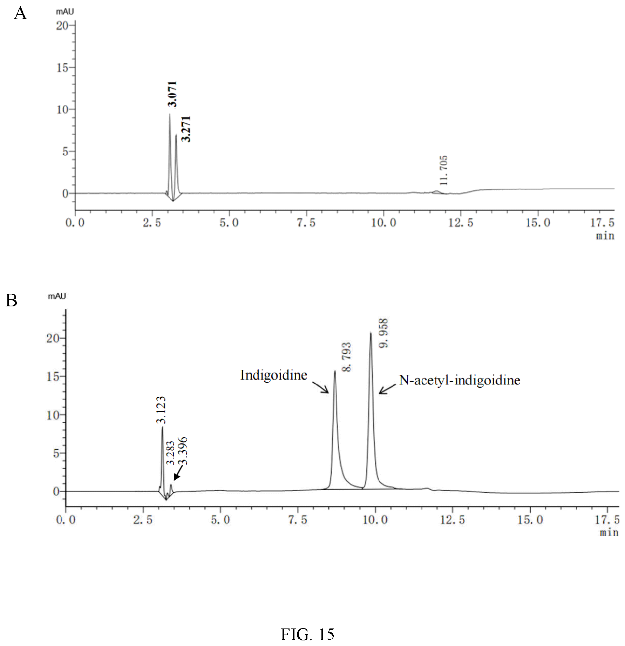

Testing results are as shown in FIG. 15; FIG. 15-A shows testing results of streptomyces lividans TK24, and there are no significant peaks at 8.8 min and 9.9 min; FIG. 15-B shows testing results of the HG-N-Idg05 strain, with one peak each present at around 8.8 min and 9.9 min, which is consistent with the appearance time of indigoidine and N-acetyl-indigoidine, respectively. It is indicated that the original strain TK24 is unable to catalyze glutamine and N-acetyl-indigoidine to synthesize N-acetyl-indigoidine, the recombinant Streptomyces lividans HG-N-Idg05 overexpresses *bpsA* and *entD* genes by plasmids to achieve catalysis of glutamine and N-acetylglutamine to synthesize N-acetyl-indigoidine.

The present application achieves catalytic synthesis of N-acetyl-indigoidine from glutamine and N-acetylglutamine in Escherichia coli, Corynebacterium glutamicum, Saccharomyces cerevisiae and Streptomyces.

### Comparative Example 1. Color comparison of DMSO solutions of indigoidine and N-acetyl-indigoidine

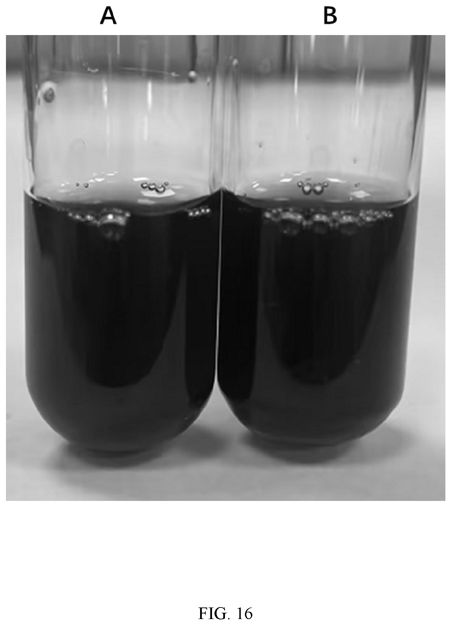

1 mg of standard substance of indigoidine is weighed, 10 mL of DMSO is added to resuspend precipitate, meanwhile, 1 mg of N-acetyl-indigoidine purified in Example 4 is weighed, and 10 mL of DMSO is added to resuspend precipitate. The two sets of results are observed for differences in color, and the results are as shown in FIG. 16; FIG. 16-A is for the DMSO solution of N-acetyl-indigoidine, and the solution is bright blue; FIG. 16-B is for the DMSO solution of the indigoidine, and the solution is blue. The color of the DMSO solution of N-acetyl-indigoidine is brighter and more vivid than that of the DMSO solution of indigoidine.

### Comparative Example 2. Color comparison of aqueous solutions of indigoidine and N-acetyl-indigoidine

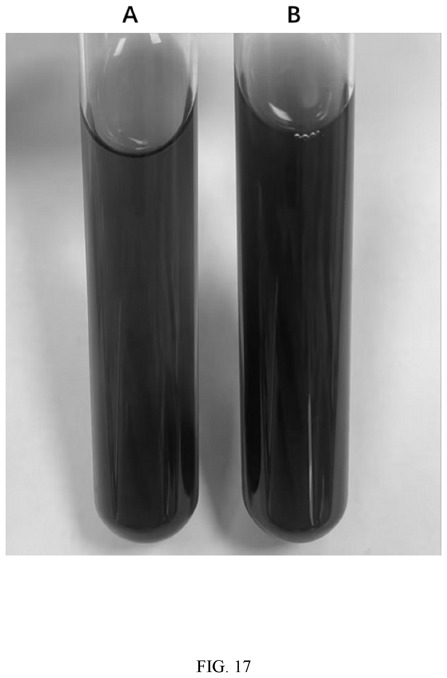

1 mg of standard substance of indigoidine is weighed, 10 mL of ddH₂O is added to resuspend precipitate, meanwhile, 1 mg of N-acetyl-indigoidine purified in Example 4 is weighed, and 10 mL of ddH₂O is added to resuspend precipitate. The two sets of results are observed for differences in color, and the results are as shown in FIG. 17; FIG. 17-A is for the aqueous solution of N-acetyl-indigoidine, which is blue; FIG. 17-B is for the aqueous solution of indigoidine, which is blue-purple; the color of N-acetyl-indigoidine is purer.

Finally, it should be noted that the foregoing examples are merely intended to describe the technical solutions of the present application, but not intended to constitute any limitation. Although the present application is described in detail with reference to preferred embodiments, it will be understood by those of ordinary skill in the art that modifications or equivalent substitutions may be made to the technical solutions of the present application without departing from the essence and scope of the technical solutions of the present application.

## Claims

1. A natural blue pigment, wherein the natural blue pigment has a chemical name of N-acetyl-indigoidine, a molecular formula of C₁₂H₁₀N₄O₅, and a chemical structure as shown in formula I;

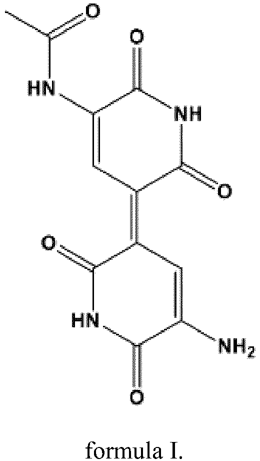

2. A biosynthesis method for the natural blue pigment according to claim 1, wherein the biosynthesis method uses metabolically engineered bacteria to express indigoidine synthetase and phosphopantetheinyl transferase, to catalyze glutamine and N-acetylglutamine to biosynthesize the natural blue pigment.

3. The biosynthesis method for the natural blue pigment according to claim 2, wherein an encoding gene of the indigoidine synthetase comprises an indigoidine synthetase encoding gene *bpsA* or an indigoidine synthetase encoding gene *indC*;
an encoding gene of the phosphopantetheinyl transferase comprises a phosphopantetheinyl transferase encoding gene *EntD* or a phosphopantetheinyl transferase encoding gene *indB.*

4. The biosynthesis method for the natural blue pigment according to claim 2, wherein the metabolically engineered bacteria are:
metabolically engineered bacteria constructed by introducing the indigoidine synthetase encoding gene *bpsA* and the phosphopantetheinyl transferase encoding gene *EntD* into host bacteria;
or,
metabolically engineered bacteria constructed by introducing the indigoidine synthetase encoding gene *indC* and the phosphopantetheinyl transferase encoding gene *indB* into host bacteria.

5. The biosynthesis method for the natural blue pigment according to claim 4, wherein a method for constructing the metabolically engineered bacteria comprises the following steps:
subjecting the indigoidine synthetase encoding gene *bpsA* and the phosphopantetheinyl transferase encoding gene *EntD* to PCR amplification and ligation into plasmids, respectively, to obtain the metabolically engineered bacteria;
or, subjecting the indigoidine synthetase encoding gene *indC* and the phosphopantetheinyl transferase encoding gene *indB* to PCR amplification and ligation into plasmids, respectively, to obtain the metabolically engineered bacteria.

6. The biosynthesis method for the natural blue pigment according to claim 5, wherein the plasmids comprise at least one of pCDFDuet, pXMJ19, pRS425, and pKC1139 plasmids.

7. The biosynthesis method for the natural blue pigment according to claim 5, wherein the PCR amplification is performed using primers with a sequence as shown in SEQ ID NO: 5 to 30.

8. The biosynthesis method for the natural blue pigment according to claim 4, wherein the host bacteria comprise at least one of *Escherichia coli*, *Corynebacterium glutamicum*, *Saccharomyces cerevisiae* and *Streptomyces lividans*.

9. The biosynthesis method for the natural blue pigment according to claim 4, wherein the biosynthesis method comprises: collecting bacterial cells induced the cultivation of the metabolically engineered bacteria by centrifugation, resuspending the bacterial cells using a catalytic solution containing phosphate buffer, glutamine, N-acetylglutamine, obtaining a transformation solution after catalysis, and collecting the natural blue pigment in the transformation solution by centrifugation.

10. The biosynthesis method for the natural blue pigment according to claim 9, wherein a concentration of glutamine in the catalytic solution is 0.1-10 g/L, and a concentration of N-acetylglutamine is 0.1-10 g/L.

11. A cellular catalyst, containing the metabolically engineered bacteria according to claim 4 or 5.
